# EUROPÄISCHE PATENTSCHRIFT

(11) **EP 2 750 645 B1**
(45) Veröffentlichungstag und Bekanntmachung des Hinweises auf die Patenterteilung: **09.08.2017**
(21) Anmeldenummer: 12798207.2
(22) Anmeldetag: 20.11.2012
(51) Int. Cl.: A61F 13/08, A61H 9/00

(54) **MEDIZINISCHER STÜTZ- UND KOMPRESSIONSSTRUMPF**
MEDICAL SUPPORT AND COMPRESSION STOCKING
BAS MÉDICAL DE SOUTIEN ET DE COMPRESSION

(30) Priorität: 21.11.2011 CH 18622011
(43) Veröffentlichungstag der Anmeldung: 09.07.2014
(73) Patentinhaber: Eidgenössische Materialprüfungs- und Forschungsanstalt EMPA, 8600 Dübendorf (CH); Sigvaris AG, 9014 St. Gallen (CH); UNICO swiss tex GmbH, 6053 Alpnachstad (CH); MSR Electronics GmbH, 8444 Henggart (CH)
(72) Erfinder: BERTAUX-HEGEMANN, Emilie, 9014 St. Gallen (CH); EGLI, Wendelin, 8472 Seuzach (CH); HESS, Markus, 6053 Alpnachstad (CH); LEHMEIER, Frederike, 9014 St. Gallen (CH); MISSAGIA, Lino, 9014 St. Gallen (CH); WEDER, Markus, 9014 St. Gallen (CH)
(74) Vertreter: Schmauder & Partner AG Patent- & Markenanwälte VSP
(86) Internationale Anmeldenummer: PCT/EP2012/073133
(87) Internationale Veröffentlichungsnummer: WO 2013/076096

(56) Entgegenhaltungen:
- DE-A1-102006 048 313
- US-A- 2 747 570
- US-A- 3 826 249
- US-A- 4 153 050
- US-A1- 2006 036 203
- US-A1- 2009 177 222
- US-A1- 2009 234 262
- US-A1- 2010 268 130
- US-A1- 2011 082 401

## Beschreibung

### Technisches Gebiet

Die Erfindung betrifft einen medizinischen Stütz- und Kompressionsstrumpf.

### Stand der Technik

Stütz- und Kompressionsstrümpfe bzw. ähnliche Hilfsmittel sind - unter anderem aus der EP 2 138 140 A2, DE 10 2008 029 289 A1, DE 10 2006 048 313 A1, DE 199 44 030 C1 - seit langem bekannt.

Medizinische Kompressionsstrümpfe werden heute speziell bei älteren Leuten bei Venenleiden eingesetzt. Um Folgeerkrankungen wie z.B. Thrombosen vorzubeugen, sollten diese Strümpfe möglichst oft getragen werden. Ein wesentliches Problem ist jedoch das An- und Ausziehen bei einfachen elastischen Stütz- und Kompressionsstrümpfen. Wegen der relativ hohen Kompression, die im Bereich von 15 bis über 49 mm Hg liegt, muss der elastische Strumpf beim Anziehen stark verdehnt werden was eine starke Kraftanstrengung seitens des Trägers des Stütz- und Kompressionsstrumpfes bedarf. Aus diesem Grund ist teilweise ein fremd betreutes Handling, z.B. durch medizinische Versorgungsunternehmen bzw. gewerbliches, medizinisches Personal erforderlich, um den Strumpf an- oder auszuziehen.

Die bekannten, einfachen, elastischen Stütz- und Kompressionsstrümpfe sind in ihrer Anwendung zumeist sehr beschränkt, das heisst, dass die Kompression allerhöchstens manuell eingestellt werden kann.

Die US2747570 offenbart einen Medizinischen Stütz- und Kompressionsstrumpf mit einem gewebe- oder gewirkeartigen, im Wesentlichen schlauchförmigen Grundelement, welches so ausgelegt ist, dass es über den Unterschenkel des Trägers gezogen werden kann, zumindest einer Luftkammer, die mit Luft befüllbar ist, wobei die zumindest eine Luftkammer längs dem Stütz- und Kompressionsstrumpf schlauchförmig ausgebildet ist, das gewebe- oder gewirkeartige Grundelement weist eine Vorspannvorrichtung auf. Ein ähnlicher Stütz- oder Kompressionsstrumpf wird in US3826249 offenbart.

Aus der US 2009/234262 A1 ist ein Monitoringsystem bekannt, um den Gesundheitszustand mit irgendwelchen Sensoren z.B. auch am Bein zu erfassen. Dabei werden auch - grundsätzlich selbstverständlich bereits vorbekannte - aufblasbare Blatern erwähnt, um einen Druck im System zu erzeugen. Irgendwelche Massnahmen zur Erleichterung des Anlegens der aufblasbaren Blatern sind aber aus der US 2009/234262 A1 nicht zu entnehmen. Für ein längeres Tragen ist das Monitoringsystem der US 2009/234262 A1 weder gedacht noch geeignet, sondern vielmehr für eine stationäre Behandlung bei Lymphleiden.

Aus der US 2011/082 401 A1 ist eine Einrichtung für die Pressung in verschiedenen Sektoren ebenfalls für die Behandlung von Lymphproblemen offenbart. Der Druck wird dabei durch diverse Luftkammern angelegt. Die Kammern werden sequentiell aufgeblasen und dann miteinander wieder abgelassen, um eine Massagebewegung aufzubringen zur Drainage von Wasseransammlungen im Gewebe. Die Einrichtung der US 2011/082 401 A1 ist als Manschette ausgebildet, die über das Bein bewickelt wird. Dies erzeugt eine hohe Wärmeisolation und auch daher ist die Einrichtung für ein längeres Tragen - wie schon das Monitoringsystem der US 2009/234262 A1 - weder gedacht noch geeignet, sondern vielmehr für kurzzeitige ambulante Behandlungen.

### Darstellung der Erfindung

Aufgabe der Erfindung ist es demnach, einen verbesserten medizinischen Stütz- und Kompressionsstrumpf vorzuschlagen, bei der die beschriebenen Nachteile vom Stand der Technik zumindest vermieden werden können. Der Kompressionsstrumpf soll demnach leicht an- und ausziehbar sein und in seiner Anwendung flexibler als die einfachen elastischen Stütz- und Kompressionsstrümpfe gemäss dem Stand der Technik. Weiterhin soll der Strumpf für ein längeres Tragen geeignet sein.

Diese Aufgabe wird gelöst durch einen verbesserten medizinischen Stütz- und Kompressionsstrumpf gemäss Anspruch 1.

Es gibt verschiedene Möglichkeiten, die Wände der Luftkammer auszubilden. Erfindungsgemäss umfassen die Wände der Luftkammern luftdichte Membranen. die so ausgebildet sind, dass sie zumindest eine bestimmte Atmungsaktivität aufweisen, um zuzulassen, dass die Körperfeuchtigkeit diffundieren kann, wobei der Wasserdampfdurchgangswiderstand Ret darf nicht grösser als 20 m²Pa/W sein soll.

Der Strumpf ist vorzugsweise mit einer Elektronikeinheit ausgestattet die eine Mikropumpe mit Ventil für die Luftzufuhr, einen vorzugsweise wieder aufladbaren Akku und eine Steuerungselektronik enthält inkl. einer drahtlosen Datenübertragung, beispielsweise auf ein "Smartphone" wie vorzugsweise über eine Applikation (App) der Strumpf bedient werden kann. Dies umfasst das Einstellen der Druckwerte, das Wählen eines geeigneten Druckprofils, das Auslesen der Daten und weitere Handlungen.

Vorteilhafterweise ist der Schlauch nicht komplett rund sondern nierenförmig und kann so die Kraft besser aufbringen. Ebenso verteilt sich der Druck aufs Bein im Bereich der Luftader auf eine grössere Fläche. Der Vorteil dieser Kompressionsaufbringung liegt darin, dass praktisch stufenlos ein Druck von 5 bis 50 mm Hg aufs Bein aufgebracht werden kann und der Druck fast beliebig zeitlich verändert werden kann, um so dem Träger eine möglichst individuelle und angepasste Therapie ermöglichen zu können.

Vorteilhafterweise umfasst die Elektronikeinheit zumindest einen Drucksensor, der fortlaufend den Druck in der zumindest einen Luftkammer misst und entsprechend einem aktuellen Solldruck die Elektronik betätigt, um den Druckwert konstant oder auf einen vorgegebenen oder momentan berechneten Wert halten zu können.

Besonders vorteilhaft ist es, wenn die Elektronikeinheit zumindest einen Beschleunigungssensor umfasst, wobei die genannte Software so eingerichtet ist, dass eine bewegungsinduzierte Druckanpassung vorgesehen ist, wobei vorzugsweise in Ruhe, vornehmlich beim Sitzen oder Liegen des Trägers ein geringerer Kompressionsdruck eingestellt als beim Bewegen, wobei beim Bewegen des Trägers der Druck auf einen vorher definierten höheren Druckwert erhöhbar ist.

Der medizinische Stütz- und Kompressionsstrumpf kann als Unterschenkelbandage oder - vorteilhafterweise - mit Füsslingen ausgestattet sein. Auch kann der Strumpf entsprechende Verlängerungen für den Oberschenkel mit entsprechenden weiteren Luftkammern aufweisen. Der Strumpf kann sogar Teil einer Hose sein.

Es sollte an dieser Stelle darauf hingewiesen werden, dass "luftdicht" im Sinne der vorliegenden Erfindung bedeutet, dass der Druckverlust kleiner ist, als die Möglichkeit, Luft durch die Pumpe nachzufüllen. Typischerweise ist die Luftdichtigkeit der Kammern so gut, dass über eine Zeit von einer Stunde der Druck in der Kammer nicht wesentlich abnimmt und nahezu konstant bleibt.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können. Insbesondere ist die Anwendung auch nicht auf den engeren medizinischen Bereich eingeschränkt, sondern kann auch auf den Gebieten der Raumfahrtmedizin, der Sportmedizin etc. bei den dort betroffenen Personen erfolgen. Auch ist eine Anwendung bei der Flugthrombose-Prävention oder im Wellnessbereich möglich.

Insbesondere sollte darauf hingewiesen werden, dass bestimmte Vorteile der vorliegenden Erfindung auch ohne elektronische Einheiten erzielt werden könnten nämlich mit einem medizinischer Stütz- und Kompressionsstrumpf mit einem gewebe- oder gewirkeartigen, im Wesentlichen schlauchförmigen Grundelement, welches so ausgelegt ist, dass es über den Unterschenkel des Trägers gezogen werden kann, zumindest einer Luftkammer, die mit Luft befüllbar ist, wobei die zumindest eine Luftkammer längs dem Stütz- und Kompressionsstrumpf schlauchförmig ausgebildet ist, wenn das gewebe- oder gewirkeartigen Grundelement zumindest eine Vorspannvorrichtung, vorzugsweise zumindest einen Reissverschluss oder zumindest einen Klettverschluss aufweist, und wobei die Wände der zumindest einen Luftkammer so ausgebildet sind, dass sie zumindest eine bestimmte Atmungsaktivität aufweisen, um zuzulassen, dass die Körperfeuchtigkeit diffundieren kann, wobei der Wasserdampfdurchgangswiderstand Ret darf nicht grösser als 20 m²Pa/W ist. Die Vorspannvorrichtung kann dann so ausgebildet sein, dass der Stütz- und Kompressionsstrumpf im geschlossenen Zustand der Vorspannvorrichtung so auf dem Unterschenkel sitzt, dass der Stütz- und Kompressionsstrumpf nicht herunterrutschen kann, während der Stütz- und Kompressionsstrumpf im geöffneten Zustand der Vorspannvorrichtung leicht an den Unterschenkel angezogen und leicht vom Unterschenkel abgezogen werden kann. Vorteilhafterweise wird auch hier zumindest eine schlauchförmige Luftkammer so ausgebildet ist, dass die Luftkammer beim Aufblasen rundlich wird und sich dessen Durchmesser verkürzt bzw. beim Aufblasen nierenförmig wird. Das Aufblasen kann mit einer Handpumpe geschehen, und es kann eine mechanische Drucküberwachung vorgesehen sein. Für nichtmedizinische Anwendungen reicht es dabei aus, wenn der Träger den Druck über eine manuelle Handpumpe soweit einstellt, dass ein angenehmer Pressdruck am Bein spürbar ist.

### Kurze Beschreibung der Zeichnungen

Ausführungsbeispiele der Erfindung werden nachfolgend anhand der Zeichnungen näher beschrieben, dabei zeigen:
- Fig. 1: eine schematische Darstellung der Elemente eines medizinischen Stütz- und Kompressionsstrumpfes gemäss einem ersten Ausführungsbeispiel der Erfindung in seinem Schichtaufbau, im Grundzustand;
- Fig. 2: eine schematische Darstellung der Elemente des medizinischen Stütz- und Kompressionsstrumpfes in seinem Schichtaufbau gemäss Figur 1, im aufgepumpten Zustand;
- Fig. 3: eine schematische Darstellung der Elemente des medizinischen Stütz- und Kompressionsstrumpfes als Aufsicht gemäss Figur 1, im Grundzustand;
- Fig. 4: eine schematische Darstellung der Elemente eines medizinischen Stütz- und Kompressionsstrumpfes gemäss einem ersten Ausführungsbeispiel der Erfindung in seinem Schichtaufbau, im Grundzustand;
- Fig. 5: eine schematische Darstellung der Elemente eines medizinischen Stütz- und Kompressionsstrumpfes gemäss einer alternativen Ausführungsbeispiel der Erfindung mit einer einfachen, angebauten Fingerpumpe; und
- Fig. 6: eine schematische Darstellung der Elemente eines medizinischen Stütz- und Kompressionsstrumpfes gemäss einer weiteren alternativen Ausführungsbeispiel der Erfindung mit einer externen Handpumpe.

### Wege zur Ausführung der Erfindung

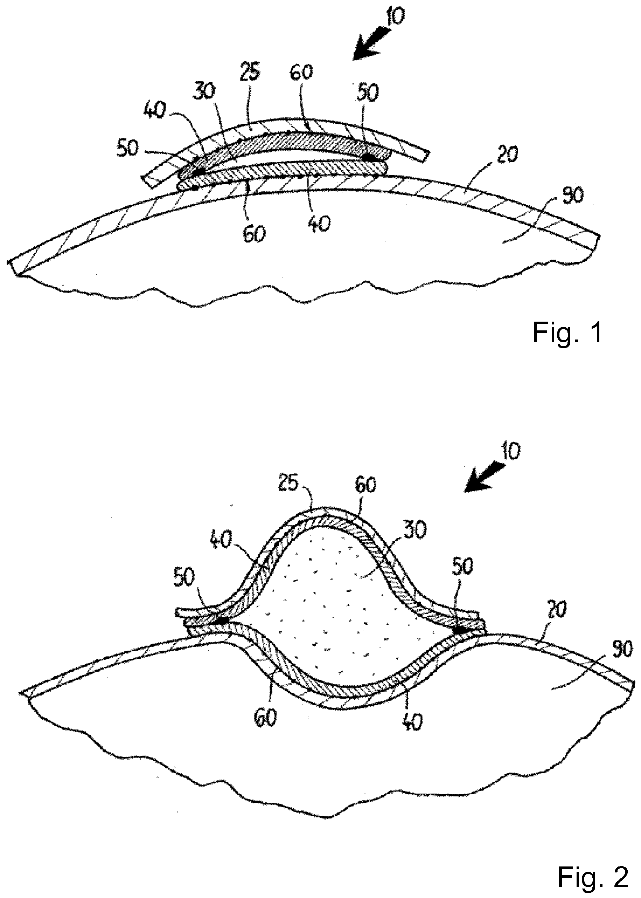

Der in Figur 1 in seinem Schichtaufbau dargestellte medizinische Stütz- und Kompressionsstrumpf 10 gemäss einem ersten Ausführungsbeispiel der vorliegenden Erfindung sieht eine erste, im wesentlichen nicht dehnfähige, gewebe- oder gewirkeartige Grundschicht 20 vor, die auf der Haut des Beines 90 des Trägers aufliegt. Die gewebe- oder gewirkeartige Grundschicht 20 bildet den eigentlichen Strumpf aus. Diese Schicht ist als Strumpf ausgebildet und hat im Ausführungsbeispiel einen Umfang, der ein einfaches Anlegen ermöglicht, also einen Umfang, der geringfügig grösser ist als die Dicke des Beines des Trägers. Sobald der Träger den Strumpf angezogen hat, kann er durch Schliessen eines spannenden Reisverschlusses (nicht dargestellt), den Strumpf fest anlegen, wobei noch keine Stützstrumpf- oder Kompressionseigenschaften im eigentlichen Sinne (gemäss den vorstehend genannten Kompressionsbedingungen mit einem Druck von 5 bis 50 mm Hg) anliegen. Im Ausführungsbeispiel ist die gewebe- oder gewirkeartige Grundschicht 20 (lediglich beispielhaft) aus PA6.6 hergestellt. Es sind aber zur Erhöhung des Tragekomforts selbstverständlich auch Schichten aus den bekannten Naturmaterialien denkbar.

Auf der gewebe- oder gewirkeartige Grundschicht 20 ist eine Luftkammer 30 vorgesehen, die mittels zweier luftdichter Membranschichten 40 gebildet wird. Im Ausführungsbeispiel ist dabei die untere Membranschicht 40 auf die gewebe- oder gewirkeartige Grundschicht 20 mittels einer Laminierung 60 auflaminiert. Die beiden luftdichten Membranschichten 40 sind an ihren Aussenkanten mittels einer Laserschweissnaht 50 luftdicht miteinander verbunden, damit eine luftdichte Luftkammer 30 gebildet werden kann.

Auf der mittels der beiden luftdichter Membranschichten 40 gebildeten Luftkammer 30, nämlich auf der äusseren Membranschicht, ist eine gewebe- oder gewirkeartige Deckschicht 25 aufgebracht, im vorliegenden Ausführungsbeispiel ebenfalls mittels einer Laminierung 60 auflaminiert. Figur 2 zeigt den Ausbau gemäss Figur 1 im aufgepumpten Zustand der Luftkammer 30. In diesem Zustand ist ein - im Ausführungsbeispiel 50 mm Hg betragender - Luftdruck in der Luftkammer 30 wegen der Eigenschaft, dass die Membranen 40 luftdicht ausgebildet und auch durch das Laserverschweissen luftdicht miteinander verbunden sind. Durch den Effekt werden die beiden Kanten der Membranen 40 bzw. der an diesen Orten mit den Membranen 40 durch Auflaminieren verbundenen Teilbereiche des Enden zusammengezogen und bewirken eine entsprechende am Bein des Trägers einwirkende Kompression und damit die gewünschte Stützeigenschaft.

Der Stütz- und Kompressionsstrumpf weist eine Drucksteuerung von 5 bis 50 mm Hg Umfangszugspannung in Relation zum Fülldruck der Luftkammern auf. Die Vorspannung des Strumpfs wird mittels Reissverschlüssen hergestellt, die nach dem anlegen geschlossen werden können. Ein in die Elektronikeinheit 70 eingebauter Drucksensor misst fortlaufend den Druck aufs Bein 90 und betätigt entsprechend die Elektronik, um den Solldruckwert möglichst konstant bzw. auf einen vorgegebenen oder momentan berechneten Wert halten zu können. Der medizinische Stütz- und Kompressionsstrumpf weist im Ausführungsbeispiel zwei Luftkammern (Luftader) auf. Die Membranen 40, welche für die Luftkammern 30 eingesetzt werden, weisen eine absolute Luftdichtigkeit auf.

Andererseits sind die Membrane bis zu einem gewissen Umfang atmungsaktiv, um die Körperfeuchtigkeit diffundieren zu können. Der Wasserdampfdurchgangswiderstand Ret soll nicht grösser als 20 m²Pa/W sein. Im hier beschriebenen Ausführungsbeispiel ist dies eine Polyetherestermembrane aus Sympatex mit einer Dicke von ca. 15 - 30 µm. Alternativ kommt eine Polyurethanmembrane aus Epurex oder einem gleichwertigen Material in Frage.

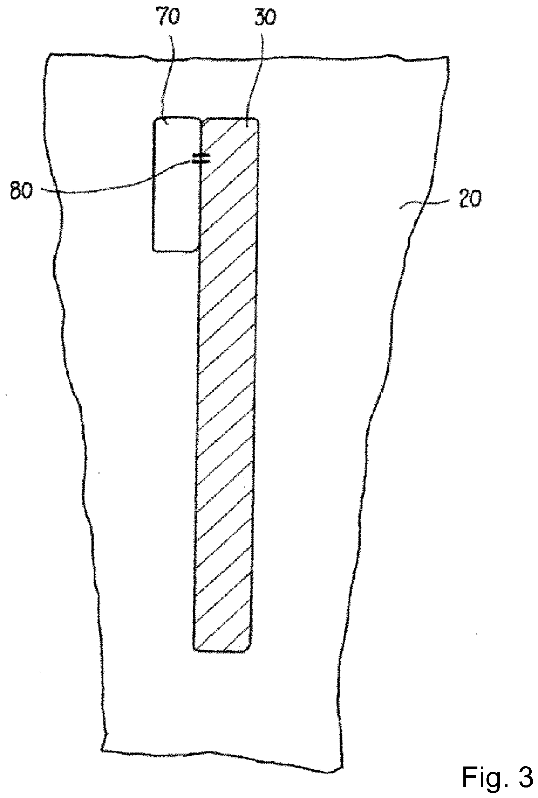

Figur 3 zeigt eine schematische Draufsicht auf den Schichtaufbau gemäss Figur 1 mit der gewebe- oder gewirkeartige Grundschicht 20 als Basis mit der als Luftader ausgebildeten Luftkammer 30 als Auflage. Die Deckschicht 25 ist in Figur 3 nicht dargestellt. An der Luftkammer 30 ist seitlich eine Elektronikeinheit 70 mit Datenlogger, Pumpe, Akku, Bewegungssensor - im vorliegenden Ausführungsbeispiel 3D-Beschleunigungssensoren - und ein Funkmodul - im vorliegenden Ausführungsbeispiel ein WiFi-Funkmodul - angeordnet und mittels eines Ventils 80 ist die Luftkammer 30 mit der Druckpumpe der Elektronikeinheit wirkverbunden, so dass sowohl Druck aufgebaut wie auch abgelassen werden kann.

Das Aufpumpen der Luftkammer bzw. Luftkammern wird mittels einer elektrischer Mikropumpe ermöglicht. Im Strumpf ist eine Elektronik mit Pumpe, Akku, 3D-Beschleunigungssensoren und Software zur Steuerung der verschiedenen Programme eingebaut. Dabei ist die Einheit so programmiert, dass eine bewegungsinduzierte Druckanpassung ermöglicht wird, wobei in Ruhe (Sitzen, Liegen) ein relativ geringer Kompressionsdruck eingestellt wird, während beim Gehen der Druck automatisch auf einen vorher definierten höheren Druckwert erhöht wird. Über den eingebauten Bewegungssensor kann ermittelt werden, was die Person gerade tut und dementsprechend kann individuell ein vorher festgelegter Kompressionsdruckwert als neuer Sollwert eingestellt werden.

Der gesamte Kompressionsstrumpf kann auch als Drucksensor verwendet werden. Dazu wird in Ruheposition ein definierter Druck eingestellt und über die Änderungen dieses Drucks bei einer körperlichen Bewegen kann die Muskelkontraktion im Unterschenkel registriert werden und über die Kurvenform des Drucks auf die Qualität des Gefässystems geschlossen werden.

Der Strumpf kann für nicht medizinische Anwendungen wie z.B. als Flugthromboseprävention, im Wellnessbereich oder im Sportbereich zur Verkürzung der Erholungsphase auch ohne Elektronik betrieben werden. Der Strumpf wird dann entweder mit einer eingebauten, einfachen Handpumpe oder einer externen Pumpe aufgepumpt bis in etwa der gewünschte Druck am Bein hergestellt ist. Ja nach Situation kann nach einer gewissen Zeit etwas nachgepumpt oder auch wieder etwas Druck abgelassen werden durch Öffnen eines mechanischen Ventils.

Der Strumpf kann auch mit Füsslingen ausgestattet sein oder auch eine ganze Hose sein, bei der auch der Oberschenkel komprimiert werden kann.

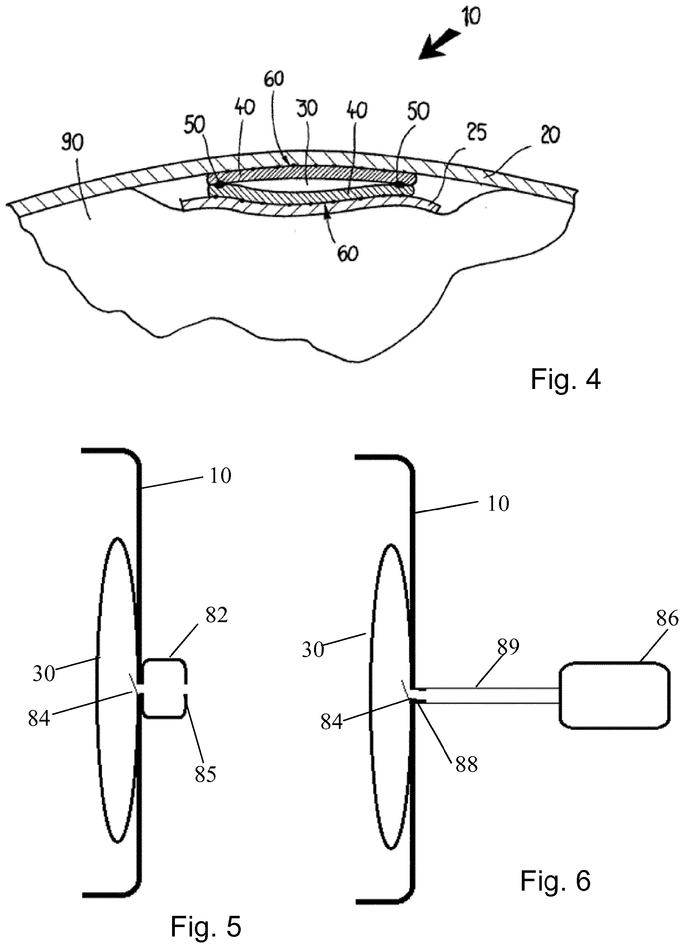

In einer alternativen Ausführung gemäss Figur 4 ist der Schichtaufbau derart geändert, dass die aus den Membranen 40 gebildete Luftkammer 30 zwischen dem Bein 90 des Trägers und der gewebe- oder gewirkeartige Grundschicht 20, die den eigentlichen Strumpf ausbildet, vorgesehen ist. Die Deckschicht 25 deckt in diesem Fall die Membranen 40 gegenüber dem Bein 90 ab. Ansonsten ist der Aufbau gleichartig zu dem Ausführungsbeispiel gemäss Figur 1 vorgesehen.

In einer weiteren alternativen Ausführung gemäss Figur 5 ist eine angebaute Fingerpumpe 82 vorgesehen. Dabei ist die Luftkammer 30 gegenüber der Fingerpumpe 82 mittels eines Ventils - im vorliegenden Ausführungsbeispiel einer Ventilklappe 84 - verschlossen und wird nur dadurch geöffnet, dass die Pumpe zusammengedrückt wird, während der Fingerverschluss 85 der Fingerpumpe durch Fingerdruck verschlossen ist.

In einer anderen alternativen Ausführung gemäss Figur 6 ist eine abnehmbare, konventionelle Pumpe 86 vorgesehen, die in der Figur ebenfalls als Handpumpe dargestellt ist. Wiederum ist die Luftkammer 30 nunmehr gegenüber dem Aussenraum 82 mittels eines Ventils - im vorliegenden Ausführungsbeispiel einer Ventilklappe 84 - verschlossen und wird nur dadurch geöffnet, dass der Einfüllstutzen 86 mit einem Überdruck gegenüber der Luftkammer 30 beaufschlagt wird. Dabei ist ein Verbindungsschlauch 89 der Handpumpe 86 mit dem Einfüllstutzen verbunden. Die "Handpumpe" 86 kann selbstverständlich wiederum als mechanisch oder elektrisch betriebene Pumpe ausgebildet sein.

### Bezugszeichenliste

- 10: Medizinischer Stütz- und Kompressionsstrumpf
- 20: gewebe- oder gewirkeartige Grundschicht
- 25: gewebe- oder gewirkeartiges Deckschicht
- 30: Luftkammer
- 40: luftdichte Membrane
- 50: Laserschweissnaht
- 60: Laminierung von der gewebe- oder gewirkeartiges Grundschicht mit der luftdichten Membrane
- 70: Elektronikeinheit mit Datenlogger, Pumpe, Akku und Funkmodul
- 80: Ventil zur Pumpe mit Drucksensor
- 82: angebaute Fingerpumpe
- 84: Ventil für Hand- oder Fingerpumpe
- 85: Fingerverschluss für die angebaute Handpumpe
- 86: externe Handpumpe
- 88: Einfüllstutzen
- 89: abnehmbarer Luftverbindungsschlauch
- 90: Bein des Trägers

## Patentansprüche

1. Medizinischer Stütz- und Kompressionsstrumpf (10) mit einem gewebe- oder gewirkeartigen, im Wesentlichen schlauchförmigen Grundelement (20), welches so ausgelegt ist, dass es über den Unterschenkel (90) des Trägers gezogen werden kann,
zumindest einer Luftkammer (30), die mit Luft befüllbar ist, wobei die zumindest eine Luftkammer (30) längs dem Stütz- und Kompressionsstrumpf (10) schlauchförmig ausgebildet ist,
**dadurch gekennzeichnet, dass** das gewebe- oder gewirkeartigen Grundelement zumindest eine Vorspannvorrichtung, nämlich zumindest einen Reissverschluss oder zumindest einen Klettverschluss aufweist, und wobei die Wände der zumindest einen Luftkammer so ausgebildet sind, dass sie zumindest eine bestimmte Atmungsaktivität aufweisen, um zuzulassen, dass die Körperfeuchtigkeit diffundieren kann, wobei der Wasserdampfdurchgangswiderstand Ret nicht grösser als 20 m²Pa/W ist und die Wände der zumindest einen Luftkammer (30) luftdichte Membranen (40) umfassen.

2. Medizinischer Stütz- und Kompressionsstrumpf nach Anspruch 1, **gekennzeichnet durch** eine Elektronikeinheit (70), die mit einer Mikropumpe für die Luftzufuhr ausgestattet ist, wobei die Mikropumpe mit einem Ventil (80) ausgestattet ist,
und eine Stromquelle, vorzugsweise einem wieder aufladbaren Akku, wobei die Elektronikeinheit (70) eine Steuerungselektronik mit einer vorzugsweise drahtlosen Datenübertragungseinheit aufweist, wobei mittels der Datenübertragungseinheit und einer in der Elektronikeinheit eingespeicherten Software die Druckwerte der zumindest einen Luftkammer einstellbar sind.

3. Medizinischer Stütz- und Kompressionsstrumpf nach Anspruch 2, **dadurch gekennzeichnet, dass** die Elektronikeinheit zumindest einen Drucksensor umfasst, der fortlaufend den Druck in der zumindest einen Luftkammer (30) misst und entsprechend einem aktuellen Solldruck die Elektronik betätigt, um den Druckwert konstant oder auf einen vorgegebenen oder momentan berechneten Wert halten zu können.

4. Medizinischer Stütz- und Kompressionsstrumpf nach einem der vorstehenden Ansprüche 1 bis 3, **dadurch gekennzeichnet, dass** die Elektronikeinheit (70) zumindest einen Beschleunigungssensor umfasst, wobei die genannte Software so eingerichtet ist, dass eine bewegungsinduzierte Druckanpassung vorgesehen ist, wobei vorzugsweise in Ruhe, vornehmlich beim Sitzen oder Liegen des Trägers ein geringerer Kompressionsdruck eingestellt als beim Bewegen, wobei beim Bewegen des Trägers der Druck auf einen vorher definierten höheren Druckwert erhöhbar ist.

5. Medizinischer Stütz- und Kompressionsstrumpf nach einem der vorstehenden Ansprüche 1 bis 4, **dadurch gekennzeichnet, dass** die Vorspannvorrichtung so ausgebildet ist, dass der Stütz- und Kompressionsstrumpf im geschlossenen Zustand der Vorspannvorrichtung so auf dem Unterschenkel sitzt, dass der Stütz- und Kompressionsstrumpf nicht herunterrutschen kann, während der Stütz- und Kompressionsstrumpf im geöffneten Zustand der Vorspannvorrichtung leicht an den Unterschenkel angezogen und leicht vom Unterschenkel abgezogen werden kann.

6. Medizinischer Stütz- und Kompressionsstrumpf nach einem der Ansprüche 1 bis 5, **gekennzeichnet durch** eine angebaute oder abnehmbare Hand- oder Fingerpumpe (82, 86).

7. Medizinischer Stütz- und Kompressionsstrumpf nach einem der Ansprüche 1 bis 6, **dadurch gekennzeichnet, dass** zumindest eine schlauchförmige Luftkammer (30) so ausgebildet ist, dass die Luftkammer beim Aufblasen rundlich wird und sich dessen Durchmesser verkürzt.

8. Medizinischer Stütz- und Kompressionsstrumpf nach einem der Ansprüche 1 bis 7, **dadurch gekennzeichnet, dass** die zumindest eine schlauchförmige Luftkammer (30) so ausgebildet ist, dass sie beim Aufblasen nierenförmig wird.

9. Medizinischer Stütz- und Kompressionsstrumpf nach einem der vorstehenden Ansprüche, **dadurch gekennzeichnet, dass** der Strumpf mit Füsslingen ausgestattet ist.

10. Medizinischer Stütz- und Kompressionsstrumpf nach einem der vorstehenden Ansprüche, **dadurch gekennzeichnet, dass** der Strumpf entsprechende Verlängerungen für den Oberschenkel mit entsprechenden weiteren Luftkammern aufweist.

11. Medizinischer Stütz- und Kompressionsstrumpf nach Anspruch 10, **dadurch gekennzeichnet, dass** der Strumpf Teil einer Hose ist.

## Claims

1. A medical support and compression stocking, (10) comprising
a woven- or knitted-fabric-type base element (20) that is substantially tubular, which is designed in such manner that it can be pulled over the lower leg (90) of the wearer,
at least one air chamber (30) that can be filled with air, wherein the at least one air chamber (30) is designed in a tubular shape along the length of the support and compression stocking (10),
**characterized in that** the woven- or knitted-fabric-type base element comprises at least one tensioning device, namely at least one zipper or at least one velcro fastener, and wherein the walls of the at least one air chamber are designed in such manner that they comprise at least a defined breathability to allow the body moisture to diffuse, wherein the water vapor transfer resistance Ret shall not exceed 20 m²Pa/W and wherein the walls of the at least one air chamber (30) comprise airtight membranes (40).

2. The medical support and compression stocking according to claim 1,
**characterized by** an electronic unit (70) that is equipped with a micropump for the air supply, the micropump being provided with a valve (80),
and by a power source, preferably a rechargeable battery, wherein the electronic unit (70) comprises control electronics including a preferably wireless data transfer unit, wherein the pressure values of the at least one air chamber can be regulated by means of the data transfer unit and a software stored in the electronic unit.

3. The medical support and compression stocking according to claim 2,
**characterized in that** the electronic unit comprises at least one pressure sensor that continuously measures the pressure in the at least one air chamber (30) and activates the electronics according to a current target pressure in order to keep the pressure value constant or on a predetermined value or currently calculated value.

4. The medical support and compression stocking according to one of the preceding claims 1 to 3, **characterized in that** the electronic unit (70) comprises at least an acceleration sensor, wherein said software is set up in such manner that a motion-induced pressure adjustment is provided, wherein a lower compression pressure is set preferably at rest, especially in the sitting or lying position of the wearer than while moving, wherein upon moving of the wearer the pressure can be increased to a previously defined higher pressure value.

5. The medical support and compression stocking according to one of the preceding claims 1 to 4, **characterized in that** the tensioning device is designed in such manner that the support and compression stocking in the closed state of the tensioning device fits on the lower leg in such manner that the support and compression stocking cannot slip down, whereas in the open state of the tensioning device the support and compression stocking can be easily put on to the lower leg and easily removed from the lower leg.

6. The medical support and compression stocking according to one of claims 1 to 5, **characterized by** an attached or detachable hand or finger pump (82, 86).

7. The medical support and compression stocking according to one of claims 1 to 6, **characterized in that** at least one tubular air chamber (30) is designed in such manner that the air chamber becomes rounded upon filling and the diameter thereof is reduced.

8. The medical support and compression stocking according to one of claims 1 to 7, **characterized in that** the at least one tubular air chamber (30) is designed in such manner that it assumes a kidney-shaped form upon filling.

9. The medical support and compression stocking according to one of the preceding claims, **characterized in that** the stocking is equipped with foot-lets.

10. The medical support and compression stocking according to one of the preceding claims, **characterized in that** the stocking comprises corresponding extensions for the upper leg with corresponding further air chambers.

11. The medical support and compression stocking according to claim 10,
**characterized in that** the stocking is part of a pair of trousers.

## Revendications

1. Bas de maintien et de contention médical (10) ayant un élément de base (20) de type tissu ou tricot essentiellement en forme de tube qui est réalisé de façon à pouvoir être tiré sur la jambe (90) du porteur,
au moins une chambre à air (30) pouvant être remplie d'air, cette chambre à air (30) étant réalisée en forme de tube le long du bas de maintien et de contention (10),
**caractérisé en ce que**
l'élément de base de type tissu ou de type tricot comprend au moins un dispositif de précontrainte, à savoir au moins une fermeture à glissoire ou au moins une fermeture auto-agrippante, et les parois de la chambre à air sont réalisées de sorte que celles-ci aient au moins une activité de respiration définie pour permettre la diffusion de l'humidité corporelle, la résistance au passage de vapeur d'eau Ret n'étant pas supérieure à 20 m²Pa/W et les parois de la chambre à air (30) comprenant des membranes (40) étanches à l'air.

2. Bas de maintien et de contention médical conforme à la revendication 1,
**caractérisé par**
une unité électronique (70) qui est équipée d'une micro-pompe d'alimentation en air, cette micro-pompe étant équipée d'une soupape (80),
et par une source de courant, de préférence un accumulateur rechargeable,
l'unité électronique (70) comprenant une électronique de commande avec une unité de transmission de données de préférence sans fil, et, l'unité de transmission de données et un logiciel enregistré dans l'unité électronique permettant de régler la valeur de la pression de la chambre à air.

3. Bas de maintien et de contention médical conforme à la revendication 2,
**caractérisé en ce que**
l'unité électronique comprend au moins un capteur de pression qui mesure en continu la pression dans la chambre à air (30) et actionne l'électronique en fonction d'une pression de consigne actuelle, pour pouvoir maintenir la valeur de la pression constante ou égale à une valeur prédéfinie ou calculée instantanément.

4. Bas de maintien et de contention médical conforme à l'une des revendications 1 à 3,
**caractérisé en ce que**
l'unité électronique (70) comprend au moins un capteur d'accélération, le logiciel étant réalisé de sorte qu'il soit prévu une adaptation de la pression induite par le mouvement, et, de préférence au repos, essentiellement lorsque le porteur est assis ou allongé est réglée une pression de compression plus faible que lors d'un mouvement, lors d'un mouvement du porteur, la pression pouvant être augmentée à une valeur de pression plus élevée définie préalablement.

5. Bas de maintien et de contention médical conforme à l'une des revendications précédentes,
**caractérisé en ce que**
le dispositif de précontrainte est réalisé de sorte que, lorsqu'il est fermé, le bas de maintien et de contention s'applique sur la jambe de façon à ne pas pouvoir glisser vers le bas, alors que, lorsqu'il est ouvert, le bas de maintien et de contention puisse facilement être tiré sur la jambe et retiré de la jambe.

6. Bas de maintien et de contention médical conforme à l'une des revendications 1 à 5,
**caractérisé par**
une pompe manuelle ou digitale (82, 86) pouvant être montée ou extraite.

7. Bas de maintien et de contention médical conforme à l'une des revendications 1 à 6,
**caractérisé en ce qu'**
au moins une chambre à air (30) en forme de tube est réalisée de sorte que lorsqu'elle est gonflée, elle devienne circulaire et que son diamètre diminue.

8. Bas de maintien et de contention médicale conforme à l'une des revendications 1 à 7,
**caractérisé en ce que**
la chambre à air en forme de tube (30) est réalisée de façon à devenir réniforme lorsqu'elle est gonflée.

9. Bas de maintien et de contention médicale conforme à l'une des revendications précédentes,
**caractérisé en ce qu'**
il est équipé de chaussons.

10. Bas de maintien et de contention médicale conforme à l'une des revendications précédentes,
**caractérisé en ce qu'**
il comporte des prolongements correspondants pour la cuisse ayant d'autres chambres à air correspondantes.

11. Bas de maintien et de contention médicale conforme à la revendication 10,
**caractérisé en ce qu'**
il constitue une partie d'un collant.
